# EUROPEAN PATENT SPECIFICATION

(11) **EP 3 333 151 B1**
(45) Date of publication and mention of the grant of the patent: **17.03.2021**
(21) Application number: 16835119.5
(22) Date of filing: 05.08.2016
(51) Int. Cl.: C07C 67/56, C07C 67/58, C07C 69/54, C07C 69/533

(54) **WATER ELIMINATION METHOD**
WASSERELIMINIERUNGSVERFAHREN
PROCÉDÉ D'ÉLIMINATION D'EAU

(30) Priority: 07.08.2015 JP 2015157786
(43) Date of publication of application: 13.06.2018
(73) Proprietor: Daikin Industries, Ltd., Osaka-shi, Osaka 530-8323 (JP)
(72) Inventor: MATSUURA, Makoto, Kita-ku Osaka-shi, Osaka 530-8323 (JP); KISHIKAWA, Yosuke, Kita-ku Osaka-shi, Osaka 530-8323 (JP); YOSHIYAMA, Asako, Kita-ku Osaka-shi, Osaka 530-8323 (JP); ISHIHARA, Sumi, Kita-ku Osaka-shi, Osaka 530-8323 (JP)
(74) Representative: Hoffmann Eitle
(86) International application number: PCT/JP2016/073207
(87) International publication number: WO 2017/026422

(56) References cited:
- DE-A1-102008 043 609
- JP-A- H07 133 252
- JP-A- 2002 251 009
- JP-A- 2004 307 586
- JP-A- 2006 151 923
- JP-A- 2011 001 340
- US-A1- 2012 059 187
- US-A1- 2015 045 579
- RYDZYNSKI ET AL: "Environmetal Health Criteria 191 - Acrylic acid", UNITED NATIONS ENVIRONMENT PROGRAMME, INTERNATIONAL LABOUR ORGANISATION; WORLD HEALTH ORGANIZATION, US, PAGE(S) 1 - 65 , 1 January 1997 (1997-01-01), XP009508718, ISBN: 978-92-4-157191-3 Retrieved from the Internet: URL:http://www.inchem.org/documents/ehc/eh c/ehc191.htm
- KOBUNSHI KAGAKU: 'CSJ: The Chemical Society of Japan' FIFTH EDITION JIKKEN KAGAKU KOZA 26 2005, pages 67 - 68, XP009508717
- 'Acrylic acid(EHC 191' IPCS INCHEM, IPCS 1997, XP009508718 ISSN: 0250-863X Retrieved from the Internet: <URL:HTTP://WWW.INCHEM.ORG/DOCUMENTS/EHC/EH C191.HTM>

## Description

### Technical Field

The present invention relates to a method for eliminating water, in particular a method for eliminating water from a composition containing an acrylic acid derivative and water.

### Background Art

Acrylic acid derivatives are widely used for e.g. materials of water-absorbing polymers, materials of acrylic resins as a substitute for inorganic glass for use in window materials for buildings and vehicles, coverings for lighting equipment, lantern signs, road signs, daily necessities, office supplies, crafts, windscreens of watches, and acrylic resin coating materials. Fluorine-containing acrylic derivatives are useful as synthetic intermediates of pharmaceuticals (e.g., antibiotics), synthetic intermediates for sheath materials of optical fibers, synthetic intermediates of coating materials, synthetic intermediates of semiconductor resist materials, and monomers of functional polymers.

Examples of known methods for producing an acrylic acid derivative include a method of producing an acrylic acid derivative by oxidizing isobutylene or propylene, and a method of producing an acrylic acid derivative using ethylene or propyne as a starting material using a transition metal catalyst.

To produce a fluorine-containing acrylic acid derivative, US-A-2012/059187 (JP-A-2011-001340) discloses, for example, a method of reacting a 2-fluoropropionic ester with a nitrogen-bromine-bond-containing brominating agent in the presence of a radical initiator, and JP-A-2012-530756 discloses a process for converting a 3-halo-2-fluoropropionic acid derivative to a substituted 2-fluoroacrylic acid derivative in the presence of at least one kind of base and at least one kind of polymerization inhibitor.

US-A-2015/045579 discloses a method for separating methyl methacrylate from a mixture containing methyl methacrylate, methanol and water, comprising the steps of:
(i) dehydrating the mixture using a first stage membrane unit comprising a hydrophilic membrane which is at least one of (a) Na₁₂Al₁₂Si₁₂O₄₈·27H₂O, (b) Si_{0.01-0.98}Al_{0.01-0.60}P_{0.01-0.52}O₂ and (c) microporous aluminophosphates, to produce a dehydrated mixture containing methyl methacrylate and methanol; and
(ii) removing methanol from the dehydrated mixture using a second stage membrane unit comprising a hydrophilic membrane having a faujasite structure.

DE-A-10 2008 043609 describes a method for producing water-free (meth)acrylic acid by removing water by treating with an adsorptive drying agent, such a molecular sieve, and/or by reacting with a reagent, such as (meth)acrylic acid anhydride or a high boiling acid, such as sulfuric acid or phosphoric acid.

### Summary of Invention

### Technical Problem

In the production of an acrylic acid derivative, water can undesirably coexist in a target acrylic acid derivative-containing composition.

Acrylic acid derivatives are hydrolyzed with water, which may possibly adversely affect the stability of the acrylic acid derivatives.

Therefore, more specifically, even a trace amount of water may possibly cause an adverse effect on desired reactions when an acrylic acid derivative is used, for example, in the applications mentioned above, i.e., synthetic intermediates of pharmaceuticals (e.g., antibiotics), synthetic intermediates for sheath materials of optical fibers, synthetic intermediates of coating materials, synthetic intermediates of semiconductor resist materials, and monomers of functional polymers.

Further, even a trace amount of water may also possibly cause an adverse effect on the preservability of acrylic acid derivatives.

Accordingly, the development of a method for efficiently eliminating water from a composition containing an acrylic acid derivative and water has been in demand.

An object of the present invention is to provide a method for efficiently eliminating water from a composition containing an acrylic acid derivative and water.

### Solution to Problem

As a result of extensive research, the present inventors found a method that can solve the above problems, and the present invention has thus been accomplished. Hence, the present invention provides a method for eliminating water from a composition A containing:
(A) an acrylic acid derivative of formula (I): 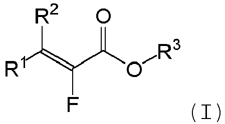 wherein
   R¹, R² each independently are H, halogen, alkyl, fluoroalkyl or optionally substituted aryl, and
   R³ is H, alkyl, fluoroalkyl or optionally substituted aryl; and
(B) water,
   the method comprises contacting the composition A with a zeolite, wherein the composition A is an organic phase obtained by washing with water a composition B containing the acrylic acid derivative of formula (I) and at least one member selected from alcohols and aldehydes as a water-soluble impurity, and eliminating an aqueous phase generated by the washing.

Also, the present invention provides a composition containing:
(A) an acrylic acid derivative of formula (I): 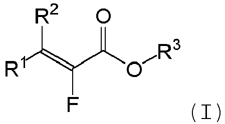 wherein
   R¹, R² each independently are H, halogen, alkyl, fluoroalkyl, or optionally substituted aryl, and
   R³ is H, alkyl, fluoroalkyl or optionally substituted aryl; and
(B) 1,000-20,000 ppm (w/w) of water.

Preferred embodiments of the invention are as defined in the appended dependent claims and/or in the following detailed description.

### Advantageous Effects of Invention

An object of the present invention is to provide a method for efficiently eliminating water from a composition containing an acrylic acid derivative and water.

### Description of Embodiments

### Terms

In this specification, terms and definitions apply. "Room temperature" refers to a temperature of 10-40°C.

The symbols and the abbreviations are to be interpreted as having the general meanings in the related technical field to which the present invention pertains, according to the context of this specification, unless otherwise specified.

The term "comprise/contain" encompasses the meanings of "consist essentially of" and "consist of."

"Alkyl" may be cyclic, linear, or branched, and may be, for example, C₁₋₂₀, C₁₋₁₂, C₁₋₆, C₁₋₄, or C₁₋₃ alkyl.

Specific examples of "alkyl" include linear or branched alkyl groups such as methyl, ethyl, propyl, isopropyl, butyl, isobutyl, sec-butyl, tert-butyl, pentyl, neopentyl and hexyl, and include C₃₋₆ cyclic alkyl (cycloalkyl)such as cyclopropyl, cyclobutyl, cyclopentyl and cyclohexyl.

"Fluoroalkyl" refers to alkyl in which at least one H is replaced by fluorine, and "fluoroalkyl" may be linear or branched.

The number of fluorine atoms in the "fluoroalkyl" may be one or more (e.g., 1-3, 1-6, 1-12, or 1 to the maximum replaceable number).

Examples of "fluoroalkyl" include C₁₋₂₀, C₁₋₁₂, C₁₋₆, C₁₋₄, and C₁₋₃ fluoroalkyl groups, and specific examples include fluoromethyl, difluoromethyl, trifluoromethyl, 2,2,2-trifluoroethyl, pentafluoroethyl, tetrafluoropropyl (e.g., HCF₂CF₂CH₂-), hexafluoropropyl (e.g., (CF₃)₂CH-), nonafluorobutyl, octafluoropentyl (e.g., HCF₂CF₂CF₂CF₂CH₂-) and tridecafluorohexyl.

Examples of "aryl" include phenyl and naphthyl, and examples of "halogen" include fluorine, chlorine, bromine, and iodine.

### Water Elimination Method

The method of the invention is a method for eliminating water from composition A containing:
(A) an acrylic acid derivative of formula (I): 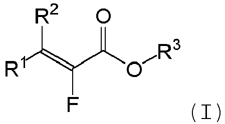 wherein
   R¹, R² each independently are H, halogen, alkyl, fluoroalkyl or optionally substituted aryl, and
   R³ is H, alkyl, fluoroalkyl or optionally substituted aryl; and
(B) water,
   the method comprises contacting the composition A with a zeolite, wherein the composition A is an organic phase obtained by washing with water a composition B containing the acrylic acid derivative of formula (I) and at least one member selected from alcohols and aldehydes as a water-soluble impurity, and eliminating an aqueous phase generated by the washing.

Composition A to be subjected to the water elimination method of the present invention ("composition A" hereinafter) may be acrylic acid derivative (A) containing water as an impurity.

More specifically, one embodiment of the invention encompasses a method for purifying acrylic acid derivative (A), the method comprising bringing a roughly purified product of acrylic acid derivative (A) containing water as an impurity into contact with a zeolite to eliminate the water.

Acrylic acid derivative (A) may be a compound to be purified by a purification method comprising the method of the present invention.

In Formula (I), R¹ is preferably H, C₁₋₂₀ (preferably C₁₋₁₂, more preferably C₁₋₆, further preferably C₁₋₄, further more preferably C₁₋₃, particularly preferably C₁ or C₂) alkyl, or C₁₋₂₀ (preferably C₁₋₁₂, more preferably C₁₋₆, further preferably C₁₋₄, further more preferably C₁₋₃, particularly preferably C₁ or C₂) fluoroalkyl, and more preferably H.

In Formula (I), R² is preferably H, C₁₋₂₀ (preferably C₁₋₁₂, more preferably C₁₋₆, further preferably C₁₋₄, further more preferably C₁₋₃, particularly preferably C₁ or C₂) alkyl, or C₁₋₂₀ (preferably C₁₋₁₂, more preferably C₁₋₆, further preferably C₁₋₄, further more preferably C₁₋₃, particularly preferably C₁ or C₂) fluoroalkyl, and more preferably H.

In Formula (I), R³ is preferably C₁₋₂₀ (preferably C₁₋₁₂, more preferably C₁₋₆, further preferably C₁₋₄, further more preferably C₁₋₃, particularly preferably C₁ or C₂) linear alkyl, more preferably methyl or ethyl, and further more preferably methyl.

In Formula (I), R¹ is preferably H, R² is preferably H, and R³ is preferably methyl or ethyl (and more preferably methyl.

Acrylic acid derivative (A) may be produced by a known method, or a method similar thereto. Alternatively, acrylic acid derivative (A) is commercially available.

Acrylic acid derivative (A) may be produced, for example, through the production method disclosed in WO 2014/034906, or a method similar thereto.

Composition A is preferably a liquid.

In composition A , acrylic acid derivative (A) is preferably mixed with water. That is, composition A is preferably of a single phase in which a phase containing acrylic acid derivative (A) is not separated from a phase containing water.

In composition A, the lower limit of the water content is preferably 1000 ppm (w/w), more preferably 2000 ppm (w/w), and further more preferably 3000 ppm (w/w), and the upper limit thereof is preferably 20000 ppm (w/w), more preferably 15000 ppm (w/w), and further more preferably 10000 ppm (w/w).

The water content in composition A is preferably 1000-20000 ppm (w/w), more preferably 2000-15000 ppm (w/w), and further more preferably 3000-10000 ppm (w/w).

In composition A, the lower limit of the acrylic acid derivative (A) content may be, but is not limited to, for example, 85% (w/w), 90% (w/w), or 95%(w/w), and the upper limit thereof may be, but is not limited to, for example, 90% (w/w), 95% (w/w), or 99% (w/w).

In composition A, the lower limit of the amount ratio of water/acrylic acid derivative (A) is preferably 1000 ppm (w/w), more preferably 1050 ppm (w/w), and further more preferably 1100 ppm (w/w), and the upper limit thereof is preferably 25000 ppm (w/w), more preferably 18000 ppm (w/w), and further more preferably 11000 ppm (w/w).

The amount ratio of water/acrylic acid derivative (A) in composition A is preferably 1000-25000 ppm (w/w), more preferably 1050-18000 ppm (w/w), and further more preferably 1100-11000 ppm (w/w).

Composition A may contain one or more other substances, in addition to acrylic acid derivative (A) and water.

The zeolite used in the present method of the invention ("the zeolite" hereinafter) may be a natural zeolite or a synthetic zeolite.

The zeolite is preferably, for example, a synthetic zeolite.

The zeolite used is preferably represented by the formula M_{2/n}O•Al₂O₃•_{X}SiO₂•yH₂O (M is a metal cation, n is its atomic valence, x is a coefficient, and y is a coefficient). M is preferably at least one metal cation selected from sodium cation and potassium cation.

The zeolite is preferably represented by the formula Na₁₂[(AlO₂)₁₂(SiO₂)₁₂]•27H₂O.

The zeolite is preferably porous.

The zeolite preferably has an average pore size of 0.3-0.5 nm (3-5 Å), preferably 0.3-0.4 nm (3-4 Å).

Such zeolite is commercially available. Specific examples include molecular sieves 3A, 4A, and 5A (Union Showa K.K.), and Zeolum 3A and 4A (Tosoh Corporation).

The zeolite is preferably, for example, molecular sieve 3A or molecular sieve 4A, and more preferably, for example, molecular sieve 4A.

The zeolite may be in the form of, for example, powder, granule, or pellets, and preferably powder or granule.

The zeolite preferably has a weight average particle size of ≤ 10 µm, and more preferably ≤ 5 µm. The particle size as used herein refers to the major axis. In this specification, when zeolite primary particles constitute secondary particles, the term "weight average particle size" refers to the particle size of the secondary particles.

In the present method, the zeolite may be of a single kind, or a combination of two or more kinds.

The zeolite may be subjected to activation treatment before use.

The conditions for the activation treatment may be, for example, a dry treatment in which heating is performed overnight at a temperature of 300-350°C in vacuum (13.33-0.13 Pa (10⁻¹ to 10⁻³ mmHg)) .

In the present method, a zeolite that has not been subjected to such an activation treatment can also be suitably used.

The amount of zeolite used in the present method is preferably, for example, 0.1-50 parts by mass (pbm), more preferably 0.3-40 pbm, and further more preferably 0.5-30 pbm, per 100 pbm of the water contained in composition A.

In the present method, the method for bringing composition A into contact with a zeolite is not particularly limited as long as composition A comes into contact with the zeolite. This method may be performed batch-wise, or continuously. Examples of batch-wise methods include a method comprising placing a zeolite into a container containing composition A, optionally stirring the mixture, and after a defined period of time, eliminating the zeolite by e.g. filtration. Examples of the continuous methods include a method comprising allowing compound A to pass through a zeolite-containing column.

In the present method, the temperature at which composition A is brought into contact with a zeolite is, for example, -10 to 50°C or 0-40°C.

In the present method, the temperature at which composition A is brought into contact with a zeolite may be room temperature.

In the present method, the time for composition A to be in contact with a zeolite is appropriately set to be sufficient to enable desirable elimination of water. Specifically, for example, in a batch-wise method, the times is usually ≥ 1 minute, and, for example, 0.1-5 hours or 0.3-2.5 hours.

Composition A used in the present method is an organic phase obtained by washing with water composition B containing (A) an acrylic acid derivative of formula (I) as defined above; and a water-soluble impurity, and eliminating the aqueous phase generated by the washing.

The "water-soluble impurity" as used herein may be a substance that has solubility in water to a degree such that the impurity can be eliminated by water-washing under common conditions.

Examples of such a water-soluble impurity include alcohols, such as methanol, ethanol, and propanol; and aldehydes, such as formaldehyde. These impurities may be used alone, or in a combination of two or more.

The total amount of the water-soluble impurity contained in composition A that is an organic phase obtained by water-washing above, is preferably ≤ 3% (w/w), and more preferably ≤ 1% (w/w).

In composition A from which all or some of the water was eliminated by using the present method (this composition may sometimes be referred to as "composition A'") the upper limit of water content may be, for example, in units of ppm (w/w), 2000, 1800, 1600, 1400, 1200, 1000 or 800 and the lower limit thereof may be, for example, in units of ppm (w/w), 100, 200, 300, 400, 500 or 600.

In one embodiment of the present invention, the numerical value of the water content can serve as the amount ratio of water/composition A'.

In one embodiment of the present invention, the numerical value of the water content can serve as the amount ratio of water/acrylic acid derivative (A).

### Examples

The present invention is described below in more detail with reference to Examples.

The following are the meanings of the symbols and abbreviations used in the Examples. In addition, symbols and abbreviations usually used in the related technical field to which the present invention pertains may be used in this specification.

In the following Examples, the water content was measured by a Karl Fischer moisture meter, the amount of methanol was measured by gas chromatography, and the amounts of methyl methacrylate and 2-fluoroacrylic acid methyl ester were measured by gas chromatography.

### Example A

### Example A1

A sample of methyl methacrylate containing 5100 ppm (w/w) of water was prepared. Then, 5 wt% of a molecular sieve (MS4A (powder), Union Showa K.K.) was added to the sample, followed by stirring for 0.5 h. The water content in the sample after stirring was 1350 ppm (w/w) (i.e., the water elimination rate was 73.5%).

### Example B

### Water-Washing (Elimination of Methanol from Methanol-Containing Sample (Preparation of Water-Containing Sample))

A sample in which the amount ratio of methanol/2-fluoroacrylic acid methyl ester was 41.5% (w/w) was prepared (sample before water-washing).

The sample (sample before water-washing) was washed with 2.0-fold mass of water.

The methanol content in the sample after water-washing as the amount ratio of methanol/2-fluoroacrylic acid methyl ester was 0.53% (w/w).

The recovery of 2-fluoroacrylic acid methyl ester was 67.8%.

The water content in the sample after water-washing was 4900 ppm (w/w).

### Comparative Examples B1 and B2, Examples B1 and B2 (Elimination of Water)

Samples of 2-fluoroacrylic acid methyl ester containing water in the amounts shown in Table 1 were prepared by using a method similar to the water-washing above. As shown in Table 1, the samples had different water contents. The difference is within the possible range among different preparation lots. The methanol content in each sample as the amount ratio of methanol/2-fluoroacrylic acid methyl ester was 0.53% (w/w).

MgSO₄ as a dehydrating agent and molecular sieve 4A (MS-4A (powder), Union Showa K.K.) or molecular sieve 3A (MS-3A (powder), Union Showa K.K.) were added at an amount ratio of 5% (w/w) to each sample, and the mixtures were gently stirred for 5 or 2 hours. Thereafter, the samples from which the dehydrating agent was eliminated were obtained by filtration, followed by measurement of water contents and calculation of water reduction rates. Table 1 shows the results.

**Table 1**

| | Dehydrating agent | Amount of dehydrating agent added (% (w/w)) | Time (hr) | Water content before drying (ppm (w/w)) | Water content after drying (ppm (w/w)) | Water elimination rate (%) |
|---|---|---|---|---|---|---|
| Comp. Ex. B1 | MgSO₄ | 5 | 0.5 | 17010 | 5897 | 65.3 |
| Comp. Ex. B2 | MgSO₄ | 5 | 2 | 15894 | 5567 | 65.0 |
| Comp. Ex. B3 | MgSO₄ | 5 | 1 | 4384 | 3407 | 22.3 |
| Example B1 | MS-4A | 5 | 0.5 | 4900 | 659 | 86.5 |
| Example B2 | MS-3A | 5 | 2 | 7399 | 1699 | 77.0 |

The results shown in Table 1 clearly indicate that the use of a molecular sieve enabled efficient water elimination from a roughly purified product of 2-fluoroacrylic acid methyl ester containing water. However, the use of MgSO₄, which is widely used as an excellent dehydrating agent, could not efficiently eliminate water, although it showed an effect to some extent.

## Claims

1. A method for eliminating water from a composition A containing:
(A) an acrylic acid derivative of formula (I): 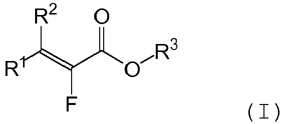 wherein
R¹, R² each independently are H, halogen, alkyl, fluoroalkyl or optionally substituted aryl, and
R³ is H, alkyl, fluoroalkyl or optionally substituted aryl; and
(B) water,
the method comprises contacting the composition A with a zeolite, wherein the composition A is an organic phase obtained by washing with water a composition B containing the acrylic acid derivative of formula (I) and at least one member selected from alcohols and aldehydes as a water-soluble impurity, and eliminating an aqueous phase generated by the washing.

2. The method of claim 1, wherein R¹ is H, C₁₋₂₀-alkyl or C₁₋₂₀-fluoroalkyl.

3. The method of claim 1 or 2, wherein R² is H, C₁₋₂₀-alkyl or C₁₋₂₀-fluoroalkyl.

4. The method of any of claims 1-3, wherein R³ is linear C₁₋₂₀-alkyl.

5. The method of any of claims 1-4, wherein the zeolite is a synthetic zeolite.

6. The method of claim 5, wherein the synthetic zeolite has an average pore size of 3-5 Å.

7. A composition containing:
(A) an acrylic acid derivative of formula (I): 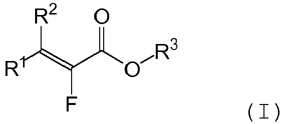 wherein
R¹, R² each independently are H, halogen, alkyl, fluoroalkyl, or optionally substituted aryl, and
R³ is H, alkyl, fluoroalkyl or optionally substituted aryl; and
(B) 1,000-20,000 ppm (w/w) of water.

## Patentansprüche

1. Verfahren zum Eliminieren von Wasser aus einer Zusammensetzung A, enthaltend:
(A) ein Acrylsäurederivat der Formel (I): 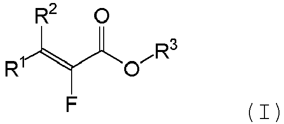 worin
R¹, R² jeweils unabhängig H, Halogen, Alkyl, Fluoralkyl oder gegebenenfalls substituiertes Aryl sind und
R³ H, Alkyl, Fluoralkyl oder gegebenenfalls substituiertes Aryl ist; und
(B) Wasser,
das Verfahren umfassend das Kontaktieren der Zusammensetzung A mit einem Zeolith, worin die Zusammensetzung A eine organische Phase ist, die durch Waschen einer Zusammensetzung B, die das Acrylsäurederivat der Formel (I) und mindestens ein Element, ausgewählt aus Alkoholen und Aldehyden, als wasserlösliche Verunreinigung enthält, mit Wasser erhalten ist, und das Eliminieren einer durch das Waschen gebildeten wässrigen Phase.

2. Verfahren gemäß Anspruch 1, worin R¹ H, C₁₋₂₀-Alkyl oder C₁₋₂₀-Fluoralkyl ist.

3. Verfahren gemäß Anspruch 1 oder 2, worin R² H, C₁₋₂₀-Alkyl oder C₁₋₂₀-Fluoralkyl ist.

4. Verfahren gemäß einem der Ansprüche 1-3, worin R³ lineares C₁₋₂₀-Alkyl ist.

5. Verfahren gemäß einem der Ansprüche 1-4, worin der Zeolith ein synthetischer Zeolith ist.

6. Verfahren gemäß Anspruch 5, worin der synthetische Zeolith eine durchschnittliche Porengröße von 3-5 Å aufweist.

7. Zusammensetzung, enthaltend:
(A) ein Acrylsäurederivat der Formel (I): 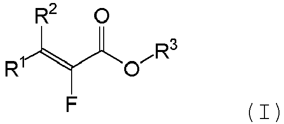 worin
R¹, R² jeweils unabhängig H, Halogen, Alkyl, Fluoralkyl oder gegebenenfalls substituiertes Aryl sind und
R³ H, Alkyl, Fluoralkyl oder gegebenenfalls substituiertes Aryl ist; und
(B) 1.000-20.000 ppm (Gew./Gew.) Wasser.

## Revendications

1. Procédé pour éliminer de l'eau d'une composition A contenant :
(A) un dérivé d'acide acrylique de formule (I) : 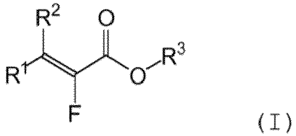 dans lequel
R¹, R² sont chacun indépendamment H, un halogène, un alkyle, un fluoroalkyle ou un aryle facultativement substitué, et
R³ est H, un alkyle, un fluoroalkyle ou un aryle facultativement substitué ; et
(B) de l'eau,
le procédé comprend la mise en contact de la composition A avec une zéolite, dans lequel la composition A est une phase organique obtenue en lavant avec de l'eau une composition B contenant le dérivé d'acide acrylique de formule (I) et au moins un élément sélectionné parmi des alcools et des aldéhydes en tant qu'impureté soluble dans l'eau et en éliminant une phase aqueuse générée par le lavage.

2. Procédé selon la revendication 1, dans lequel R¹ est H, un alkyle en C₁₋₂₀ ou un fluoroalkyle en C₁₋₂₀.

3. Procédé selon la revendication 1 ou 2, dans lequel R² est H, un alkyle en C₁₋₂₀ ou un fluoroalkyle en C₁₋₂₀.

4. Procédé selon l'une quelconque des revendications 1-3, dans lequel R³ est un alkyle linéaire en C₁₋₂₀.

5. Procédé selon l'une quelconque des revendications 1-4, dans lequel la zéolite est une zéolite synthétique.

6. Procédé selon la revendication 5, dans lequel la zéolite synthétique présente une taille de pore moyenne de 3-5 Å.

7. Composition contenant :
(A) un dérivé d'acide acrylique de formule (I) : 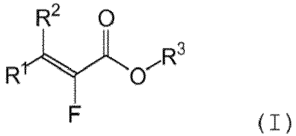 dans laquelle
R¹, R² sont chacun indépendamment H, un halogène, un alkyle, un fluoroalkyle ou un aryle facultativement substitué, et
R³ est H, un alkyle, un fluoroalkyle ou un aryle facultativement substitué ; et
(B) de 1 000 à 20 000 ppm (p/p) d'eau.
